(19) Europäisches Patentamt
European Patent Office
Office européen des brevets

(11) Publication number: **0 213 526 A1**

# (12) EUROPEAN PATENT APPLICATION

(21) Application number: 86111386.8

(22) Date of filing: 18.08.86

(51) Int. Cl.⁴: C 07 C 129/12
A 61 K 31/16

(30) Priority: 27.08.85 JP 186737/85

(43) Date of publication of application: 11.03.87 Bulletin 87/11

(84) Designated Contracting States: AT BE CH DE FR GB IT LI LU NL SE

(71) Applicant: MICROBIAL CHEMISTRY RESEARCH FOUNDATION
14-23, Kamiosaki 3 Chome Shinagawa-ku
Tokyo 141(JP)

(72) Inventor: Umezawa, Hamao, Prof.
23, Toyotama-kita 4-chome Nerima-ku
Tokyo(JP)

(72) Inventor: Takeuchi, Tomio
701A New Fuji Mansion 1-11 Higashi-gotanda 5-chome
Shinagawa-ku Tokyo(JP)

(72) Inventor: Umeda, Yoshihisa
27-21, Jinryo 2-chome
Otsu-city Shiga Pref.(JP)

(72) Inventor: Moriguchi, Makoto
52-5, Nakakitazutsumi
Joyo-city Kyoto Pref.(JP)

(72) Inventor: Kuroda, Hirosyuki
2-8, Koseicho 4-chome
Koka-gun Shiga Pref.(JP)

(72) Inventor: Nakamura, Teruya
831-6, Nomuracho
Kusatsu-City Shiga Pref.(JP)

(72) Inventor: Fujii, Akio
8-13, Ofuna 4-chome
Kamakura-city Kanagawa Pref.(JP)

(74) Representative: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) **Spergualin-related compounds, a process for their preparation and their use as medicaments.**

(57) The present invention relates to Spergualin analogues having a phenylene group and salts thereof, and to a process for their preparation.

The compounds of the invention exhibit cancer-control activity and can, therefore, be used as medicaments.

EP 0 213 526 A1

## Spergualin-related compounds, a process for their preparation and their use as medicaments

The present invention relates to Spergualin-related compounds having a phenylene group, and salts thereof, both of which are novel cancer-control substances. The present invention also relates to a process for producing such Spergualin-related compounds and salts thereof.

Spergualin is a compound which has been isolated from the filtrate of a culture of a Spergualin-producing microorganism of the genus Bacillus. Spergualin has the following structure:

16 14 12 10 8
19 15 13 11 9 7 6 5 4 3 2 1

$$H_2N\underset{\underset{NH}{\|}}{C}NH(CH_2)_4\underset{OH}{CH}CH_2CONH\underset{OH}{CH}CONHCH_2CH_2CH_2CH_2NHCH_2CH_2CH_2NH_2$$

Spergualin has the effect of inhibiting the growth of both Gram-positive and Gram-negative microorganisms. It also exhibits appreciable therapeutic and life-prolonging effects in healing experiments with mouse leukemia L-1210, mouse leukemia FL-4, Ehrlich cancer and sarcoma 180 (S-180) and has a potential for use as an anti-tumor agent (see Japanese Patent Public Disclosure No. 48975/1982).

It has been reported that Spergualin can also be obtained by synthesis techniques [see The Journal of Antibiotics, 34, 1625 (1981)].

As a result of search for Spergualin-related compounds, it has been found that superior cancer-control effects are also exhibited by compounds of the general formula V:

$$H_2N\underset{\underset{NH}{\|}}{C}NH(CH_2)_pCONH\underset{\underset{OR_3}{|}}{CH}CO\ NH(CH_2)_4NH(CH_2)_3NH_2 \qquad (V)$$

(where p is an integer of 3 - 8; and $R_3$ is a hydrogen atom, a lower alkyl group of 1 - 4 carbon atoms which may be

substituted with a hydroxyl group or a benzyl group) (Japanese Patent Public Disclosure No. 76046/1984) and by compounds of the general formula VI

$$H_2N\underset{\underset{NH}{\|}}{C}NH(CH_2)_p\underset{\underset{R_4}{|}}{C}HCH_2CO-R_5-NH(CH_2)_4NH(CH_2)_3NH_2 \quad (VI)$$

where $R_4$ is a hydrogen atom, a hydroxyl group or an aliphatic acyloxy group having 1 - 10 carbon atoms; $R_5$ is a residue obtained by eliminating both one hydrogen atom from the α- or ω-amino acid and a hydroxyl group from the carboxyl group (except for the residue of α-hydroxylglycine), with $R_5$ forming an acid amide bond with the adjacent carbonyl and amino groups; and q is an integer of 4 - 6 (Japanese Patent Public Disclosure No. 42356/1984).

The efforts to develop Spergualin-related compounds with even better cancer-control effects on the basis of the aforementioned findings, have lead to the following invention.

The present invention relates to Spergualin-related compounds having a phenylene group, the chemical structure of which is represented by the general formula I:

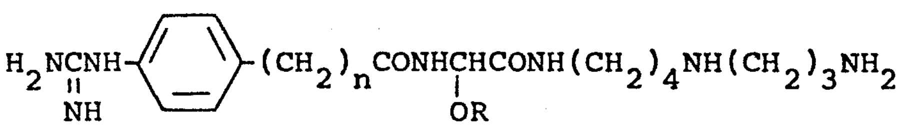

(I)

where R is a hydrogen atom or a $C_1$-$C_5$-alkyl group; and n is an integer of 3 - 5 and to salts thereof.

The present invention relates further to a process for producing a Spergualin-related compound of the formula I which comprises condensing a compound of the formula II

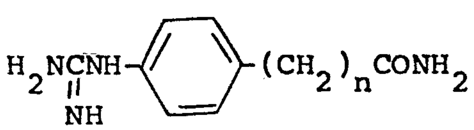

(II)

where n is as defined above, with a compound of the formula III:

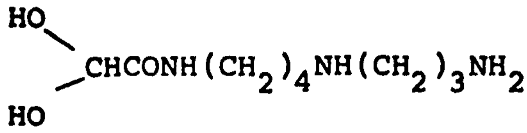

(III)

to obtain a compound of the formula I wherein R is hydrogen, and optionally reacting the compound obtained with an alcohol of the formula VI

$$R - OH \qquad (IV)$$

where R is $C_1$-$C_4$-alkyl in the presence of a catalyst to obtain a compound of the formula I wherein R is $C_1$-$C_4$-alkyl.

The compounds of the formula I exhibit pronounced therapeutic and life-prolonging effects in healing experiments with mouse leukemia L-1210, mouse leukemia FL-4, Ehrlich cancer and sarcoma 180 (S-180).

Typical examples of the compound in accordance with the first aspect of the present invention have the following physicochemical and biological properties.

(1) Physicochemical properties:

The Spergualin-related compounds of the invention are not stable when they are in the form of free bases and are preferably converted to nontoxic acid addition salts by adding pharmaceutically acceptable acids in accordance with conventional methods. The acids to be attached may be inorganic acids such as hydrochloric acid, phosphoric acid, boric acid and sulfuric acid or organic acids such as acetic acid, citric acid, tartaric acid and glutaric acid.

Salts such as hydrochlorides of these compounds are in the form of colorless hygroscopic powders and do not exhibit any definite melting points. In addition, they are racemic forms and do not show any specific rotation. The molecular formulas of hydrochlorids of typical compounds of formula I are shown in Table 1. Their IR absorption spectra (measured as KBr tablets) and proton NMR spectra (measured in d-MeOH using TMS as a reference material) are shown in Table 2.

Table 1

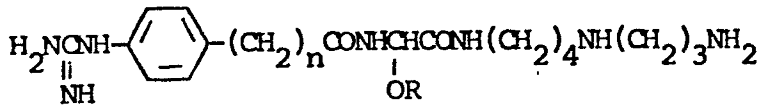

| Compound No. | n | R | Molecular formula | Name |
|---|---|---|---|---|
| 1 | 3 | H | $C_{20}H_{35}N_7O_3 \cdot 3HCl$ | 10-{N-[4-(4-guanidinophenyl)butanoyl]-α-hydroxyglycyl}-1,5,10-triazadecane |
| 2 | 3 | $CH_3$ | $C_{21}H_{37}N_7O_3 \cdot 3HCl$ | 10-{N-[4-(4-guanidinophenyl)butanoyl]-α-methoxyglycyl}-1,5,10-triazadecane |
| 3 | 5 | H | $C_{22}H_{39}N_7O_3 \cdot 3HCl$ | 10-{N-[6-(4-guanidinophenyl)hexanoyl]-α-hydroxyglycyl}-1,5,10-triazadecane |

Table 2

| Compound No. | Apsorption IR spectra (KBr) | Proton NMR spectra ($CD_3OD$) |
|---|---|---|
| 1 | 3450, 2950, 1670, 1655, 1590, 1530, 1460, 1420, 1260, 1100, | 1.6~2.3 ($CH_2$x4), 2.25 ($CH_2$), 2.7 ($CH_2$), 2.9~3.5 ($NCH_2$x4), 5.5 (CH), 7.2 (CHx2), 7.4 (CHx2) |
| 2 | 3450, 2990, 1685, 1650, 1595, 1550, 1530, 1470, 1275, 1215, 1175, 1120 | 1.6~2.3 ($CH_2$x4), 2.25 ($CH_2$), 2.68 ($CH_2$), 2.95~3.5 ($NCH_2$x4), 3.35 ($OCH_3$), 5.34 (CH), 7.2 (OHx2), 7.4 (CHx2) |
| 3 | 3460, 2970, 1675, 1650, 1580, 1525, 1465, 1420, 1270, 1100, | 1.5~2.4 ($CH_2$x6), 220 ($CH_2$), 2.7 ($CH_2$), 2.9~3.5 ($NCH_2$x4), 5.52 (CH), 7.1 (CHx2), 7.3 (CHx2) |

(2) Biological properties:

The compounds of the general formula I exhibit pronounced cancer-control effects and, as will be apparent from the data shown below, have excellent life-prolonging effects on cancer-bearing mice.

Therapeutic effects on cancer in mice

Male $BDF_1$ mice (5-wk old) in groups comprising four heads were inoculated intraperitoneally with $10^5$ cells of mouse leukemia L-1210. Starting on the day of inoculation, a selected sample dissolved in a physiological saline solution was administered intraperitoneally to each animal for 6 consecutive days on a one-dose-per-day basis. After keeping the animals for 30 days, the life-prolongation effect of sample was determined form the following formula:

Life-prolongation effect = T/C x 100 = (Average number of days of survival of the treated group / Average number of days of survival of the control group) x 100.

Table 3

| Compound No. / Dose (mg/kg/day) | 1 | | 2 | |
|---|---|---|---|---|
| | Percent life prolongation | Percent survival for 30 days | Percent life prolongation | Percent survival for 30 days |
| 50 | 0 | 0/4 | 0 | 0/4 |
| 25 | > 356 | 2/4 | > 417 | 4/4 |
| 12.5 | > 401 | 3/4 | > 417 | 4/4 |
| 6.25 | > 363 | 2/4 | > 403 | 3/4 |
| 3.13 | > 335 | 2/4 | > 368 | 3/4 |
| 1.56 | 197 | 0/4 | 171 | 0/4 |
| 0.78 | 155 | 0/4 | 118 | 0/4 |
| 0.39 | 120 | 0/4 | 111 | 0/4 |
| 0.20 | 110 | 0/4 | 104 | 0/4 |

The process for producing the compounds of the formula I is hereunder described in detail. In the Japanese Patent Public Disclosure No. 185254/1982, a method has been proposed wherein ω-guanidino aliphatic acid amide is condensed with a compound of the formula III without protecting functional groups such as guanidino and amino groups present in the compounds by any specific protective groups. The condensation of these compounds which is a dehydration reaction is preferably carried out in an anhydrous solvent. However, in consideration of the solubility of the compounds of formulas II and III which are usually handled in the form of acid addition salts, the reaction is carried out in the presence of a small amount of water. Water may be added in the necessary minimum for uniformly dissolving the compounds of formulas II and III. Higher yields are attained when the compound of formula III is used in amounts of 4 - 40 moles per mole of the compound of formula II.

Even better yields may be attained by adding acid catalysts. Illustrative acid catalysts are inorganic acids such as hydrochloric acid, sulfuric acid, phosphoric acid and boric acid, and organic acids such as acetic acid, citric acid, tartaric acid, succinic acid, glutaric acid and adipic acid. The use of carboxylic acids such as glutaric acid and citric acid is particularly preferable.

The acid is used in an amount ranging from 0 to 10 moles, preferably from 0.5 to 4 moles, per mole of the compound of formula II. The reaction temperature ranges from room temperature to 80°C, preferably from 40 to 60°C. The reaction time will vary with the temperature and preferably ranges form 5 hours to 2 days.

The starting compound of the general formula II may be synthesized from a known compound such as, for example, one having the formula:

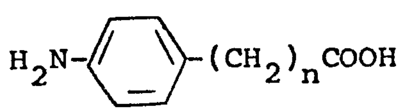

where n is as defined above, by means of a known reaction. N-[4-(3-aminopropyl)aminobutyl]-2,2-dihydroxyethane amide which is a compound of the formula III may be prepared by any of the methods elaborated in the specifications of Japanese Patent Public Disclosure Nos. 192347/1982, 52263/1983 and 104053/1985 and Japanese Patent Application No. 36263/1984.

The compounds of formula I were R= H which are used in the process of the present invention may be selected from among lower alcohols such as methanol, ethanol, propanol and butanol.

Examples of the acid catalyst that may be used include inorganic acids such as hydrochloric acid and sulfuric acid; organic acids such as acetic acid and paratoluenesulfonic acid; and cation-exchange resins such as (R)Dowex 50W (Dow Chemical Company). The reaction is carried out at temperatures ranging from room temperature to 60°C, with room temperature being preferable. The reaction period will vary with the temperature and, for attaining high yields, the reaction is preferably continued from one to several days.

Examples

The following examples are given for the purpose of further illustrating the present invention but are in no sense to be taken to limit the invention.

Example 1:

Synthesis of 10-{N-[4-(4-guanidinophenyl)butanoyl]-α-hydroxyglycyl}-1,5,10-triazadecane trihydrochloride

Four grams (15.6 mmol) of 4-(4-guanidinophenyl)butane amide hydrochloride and 5.9 g (20.2 mmol) of n-[4-(3-aminopropyl)aminobutyl]-2,2-dihydroxyethane amide dihydrochloride and 1.1 g (5.2 mmol) of succinic acid were mixed with 1.4 g of water and the mixture was heated at 60°C for 18 hours. Thereafter, 50 ml of water was added to the reaction mixture, which then was loaded onto a column

packed with 400 ml of (R)CM-Sephadex C-25 (Na form) (Pharmacia Fine Chemicals). After performing elution by a gradient of water (3.000 ml) and 1 M NaCl (3.000 ml), the active fractions were collected, concentrated under vacuum and subjected to two extractions with methanol. The extract was concentrated to 20 ml, loaded onto a column packed with 400 ml of (R)Sepharex LH-20 (Pharmacia Fine Chemicals) and eluted with methanol. The active fractions were collected and concentrated to dryness to obtain 2.7 g (5.0 mmol) of 10-{N-[4-(4-guanidinophenyl)butanoyl]-α-hydroxyglycyl}-1,5,10-triazedecane trihydrochloride in a yield of 32.4 %.

Example 2:

Synthesis of 10-{N-[4-(4-guanidinophenyl)butanoyl]-α-methoxyglycyl}-1,5,10-triazadecane trihydrochloride

Two and a half grams (4.7 mmol) of 10-{N-[4-(4-guanidinophenyl)butanoyl]-α-hydroxyglycyl}-1,5,10-triazadecane trihydrochloride was dissolved in 45 ml of methanol and, after addition of 0.7 ml of conc. HCl, the mixture was stirred for 20 hours at room temperature. The reaction solution was concentrated under vacuum and the resulting solid residue was dissolved in 500 ml of water. After its pH was adjusted to 6, the solution was loaded onto a column packed with 300 ml of (R)CM-Sephadex C-25 (Na form) and eluted by a gradient of water (3,000 ml) and 1 M NaCl (3,000 ml). The active fractions were collected, concentrated and subjected to two extractions with methanol. The extract was loaded onto a column packed with 300 ml of (R)Sephadex LH-20 and eluted with methanol. The active fractions were collected and evaporated to dryness to obtain 1.4 g (2.6 mmol) of 10-{N-[4-(4-guanidinophenyl)-butanoyl]-α-methoxyglycyl}-1,5,10-triazadecane trihydrochloride in a yield of 56.0 %.

Example 3:

Synthesis of 10-{N-[6-(4-guanidinophenyl)hexanoyl]-α-hydroxyglycyl}-1,5,10-triazadecane trihydrochloride

Three and a half grams (12.3 mmol) of 6-(4-guanidinophenyl)-hexane amide hydrochloride, 4.68 g (16 mmol) of N-(3-aminopropyl)-aminobutyl-2,2-dihydroxyethane amide dihydrochloride were mixed with 2.5 g of water and the mixture was heated at 60°C for 8 hours. Thereafter, the reaction mixture was worked up as in Example 1 to obtain 2.48 g of 10-{N-[6-(4-guanidinophenyl)hexanoyl]-α-hydroxyglycyl}-1,5,10-triazadecane trihydrochloride in a yield of 36 %.

Claims:

1. A compound of the formula I

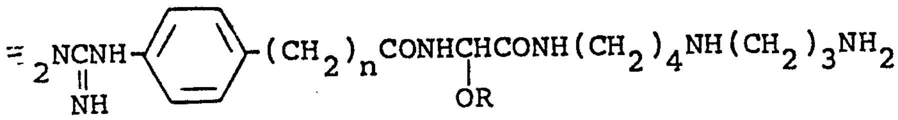

(I)

wherein R is hydrogen or $C_1$-$C_4$-alkyl and n is an integer of 3 to 5 and the physiologically acceptable salts thereof.

2. A compound as defined in claim 1 wherein R is hydrogen or methyl.

3. A process for the preparation of a compound of the formula I

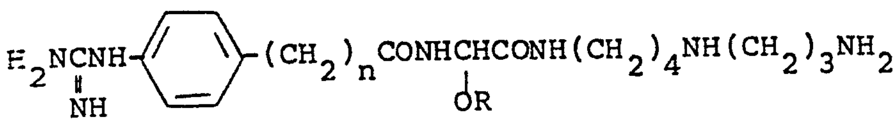

(I)

wherein R is hydrogen or $C_1$-$C_4$-alkyl and n is an integer of 3 to 5, and the physiologically acceptable salts thereof, which comprises

a) condensing a compound of the formula II

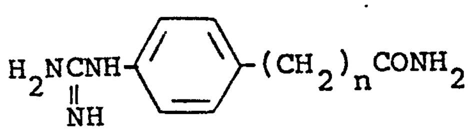

(II)

with a compound of the formula III

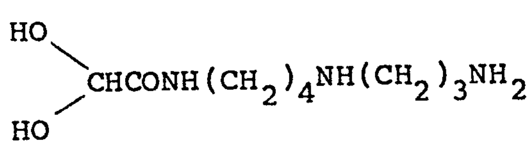

(III)

to obtain a compound of the formula I wherein R is hydrogen, and

b) optionally reacting a compound of the formula I wherein R is hydrogen, with an alcohol of the formula IV

$$R - OH \qquad (IV)$$

where R is $C_1$-$C_4$-alkyl, in the presence of a catalyst, to obtain a compound of the formula I wherein R is $C_1$-$C_4$-alkyl, and optionally preparing the physiologically acceptable acid addition salts thereof.

4. The process as defined in claim 3 wherein R is hydrogen or methyl.

5. The process as defined in claims 3 or 4 wherein the condensation reaction is conducted in the presence of an acid catalyst in an amount ranging from 0 to 10 moles per mole of the compound of formula II, at a temperature of from room temperature to 80°C and a time from 5 hours to 2 days.

6. The process as defined in claims 3 or 4, wherein the reaction according to step b) is conducted at a temperature of from room temperature to 60°C and a time from one to several days.

7. The process as defined in any of claims 3 to 5, wherein the acid catalyst is glutaric acid or citric acid.

8. A pharmaceutical composition comprising as active ingredient a compound as defined in claim 1 in association with a pharmaceutically acceptable carrier.

9. Use of a compound as defined in claim 1 for the preparation of a medicament having cancer-control activity.

Patent Claims for Austria:



1. A process for the preparation of a compound of the formula I

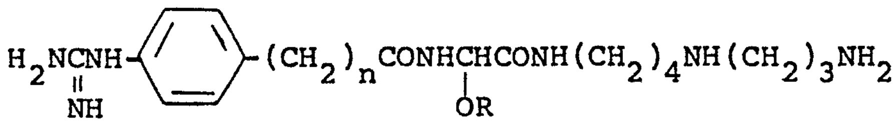 (I)

wherein R is hydrogen or $C_1$-$C_4$-alkyl and n is an integer of 3 to 5, and the physiologically acceptable salts thereof, which comprises

a) condensing a compound of the formula II

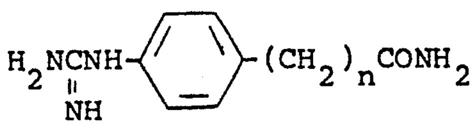 (II)

with a compound of the formula III

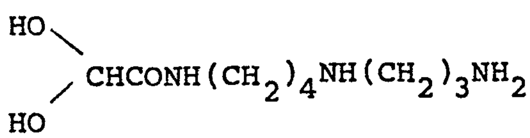 (III)

to obtain a compound of the formula I wherein R is hydrogen, and

b) optionally reacting a compound of the formula I wherein R is hydrogen, with an alcohol of the formula IV

$R - OH$ (IV)

where R is $C_1$-$C_4$-alkyl, in the presence of a catalyst, to obtain a compound of the formula I wherein R is $C_1$-$C_4$-alkyl, and optionally preparing the physiologically acceptable acid addition salts thereof.

2. The process as defined in claim 1 wherein R is hydrogen or methyl.

3. The process as defined in claims 1 or 2 wherein the condensation reaction is conducted in the presence of an acid catalyst in an amount ranging from 0 to 10 moles per mole of the compound of formula II, at a temperature of from room temperature to 80°C and a time from 5 hours to 2 days.

4. The process as defined in claims 1 or 2, wherein the reaction according to step b) is conducted at a temperature of from room temperature to 60°C and a time from one to several days.

5. The process as defined in any of claims 1 to 3, wherein the acid catalyst is glutaric acid or citric acid.

6. Use of a compound as defined in claim 1 for the preparation of a medicament having cancer-control activity.

European Patent Office

# EUROPEAN SEARCH REPORT

0213526
Application number

EP 86 11 1386

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X,P | EP-A-0 153 720 (MICROBIAL CHEMISTRY RESEARCH FOUNDATION)<br>* claims 1, 6, 7 *<br>--- | 1,8,9 | C 07 C 129/12<br>A 61 K 31/16 |
| X,P | EP-A-0 181 592 (MICROBIAL CHEMISTRY RESEARCH FOUNDATION)<br>* claims 1, 5 *<br>--- | 1,8,9 | |
| A | FR-A-2 514 350 (ZAIDAN HOJIN BISEIBUTSU KAGAKA KENKYU)<br>----- | 1 | |

TECHNICAL FIELDS SEARCHED (Int Cl 4)

C 07 C 129/12

The present search report has been drawn up for all claims

| Place of search | Date of completion of the search | Examiner |
|---|---|---|
| BERLIN | 22-10-1986 | KAPTEYN H G |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82